Europäisches Patentamt

European Patent Office

Office européen des brevets

(11) Publication number: **0 075 548**
**B1**

(12) **EUROPEAN PATENT SPECIFICATION**

(45) Date of publication of patent specification: **23.07.86**

(51) Int. Cl.⁴: **B 65 D 83/14**

(21) Application number: **82850183.3**

(22) Date of filing: **16.09.82**

(54) **Medical aerosol dispensing device.**

(30) Priority: **21.09.81 SE 8105544**

(43) Date of publication of application:
**30.03.83 Bulletin 83/13**

(45) Publication of the grant of the patent:
**23.07.86 Bulletin 86/30**

(84) Designated Contracting States:
**AT BE CH DE FR GB IT LI LU NL SE**

(56) References cited:
**GB-A-1 402 638**
**US-A-2 318 636**
**US-A-4 111 338**

(73) Proprietor: **Aktiebolaget Draco**
**Box 1707 Tunavägen 43**
**S-221 01 Lund (SE)**

(72) Inventor: **Andersson, Jan Anders Roland**
**Lundavägen 5**
**S-240 17 Södra Sandby (SE)**
Inventor: **Nyman, Sven Gösta**
**Ljungvägen 4**
**S-240 10 Dalby (SE)**
Inventor: **Snellman-Wasenius, Kaija Anneli**
**Söderkulla**
**SF-01150 Kallbäck (FI)**
Inventor: **Virtanen, Risto**
**Torenvägen 14 C**
**SF-01900 Nurmijärvi (FI)**

(74) Representative: **Wurm, Bengt Runio et al**
**Patent and Trade Mark Department AB ASTRA**
**S-151 85 Södertälje (SE)**

Courier Press, Leamington Spa, England.

## Description

### Technical field

The present invention is related to an aerosol dispensing device of the kind where an aerosol container is placed in a socket, through which an aerosol may be dispensed, and which is provided with a means protecting the dispensing opening and the aerosol container on storage. In particular, the invention is related to a device for administration of medicinally or hygienically active substances in aerosol form to the respiratory organs of a patient, primarily on administration into the nose. An object of the invention is to obtain an aerosol dispensing device having improved protection of the dispensing opening and the aerosol container. A further object is to obtain a device wherein the protective means does not incur a risk of disturbing the function of the device on aerosol dispensing.

### State of the art

Administration of medicinally or hygienically active compounds in aerosol form is employed for treatment of the nasal cavities, the oral cavity, the throat and the inner parts of the respiratory organs. The aerosol is thereby usually provided in an aerosol container placed in a socket having a spray orifice from which doses of aerosol may be dispensed. Dispensing usually takes place by activation of the valve mechanism of the container by the patient, by depressing the end of the container into the socket. In order to avoid contamination of the dispensing opening of the device, said opening has often been provided with some kind of protective lid, which may have been combined with a protective means for the end of the container, which lid concurrently prevents inadvertent dispensing. Devices having such protective means are shown in e.g. U.S. Patent Specification No. 3 429 310 and British Patent Specification No. 1 402 638. The device according to the firstmentioned specification, in which a protective cover is both turnable and axially displaceable on a cylindrical socket, is impaired by risks of disturbed function in that the cover may seize or be locked on the socket, and turning and/or depression on aerosol dispensing is prevented. The manner of use may further not be obvious to the patient. Finally, the device does not permit a sufficient flow of air therethrough on aerosol dispensing. The device according to the second specification mentioned GB—A—1 402 638 is acknowledged as the nearest prior art and has a protective lid attached at the upper end of the socket via a flexible connecting member. Said protective cover requires a considerable space in the open position and may disturb the function of the device by falling down. On aerosol inhalation through the nose the lid will be positioned close to the eyes which is inconvenient to the patient.

### Description of the invention

According to the present invention there is provided an aerosol dispensing device which fulfils the objects stated above, avoiding the drawbacks in previously known constructions and having the further advantage that the protective member, in the use position of the device, serves as a support and renders the device more secure and convenient to hold in hand.

The aerosol dispensing device according to the invention comprises a socket, said socket having an open end opening across the longitudinal direction thereof and being adapted to receive and hold an aerosol container; said socket being closed at the opposite end thereof and having, in the interior thereof, a spraying nozzle adapted to interact with a dosage valve on the aerosol container, and further having a discharge tube for an aerosol; said socket having attached thereto a protective member movable between a use position for the device and a storage position, at which the protective member covers the discharge tube as well as the open end of the socket and an aerosol container held therein; the protective member consisting of a casing (9) suspended to rotate about an axis transverse to the longitudinal direction of the socket. The device is characterised in that said casing (9) is suspended on two spindle means (7) having a common axis extending through a central part of the socket, close to a point half-way between the edges (10, 11) of the closed end and the open end of the socket, and close to a point half-way between the corresponding edges of the casing (9).

According to a preferred embodiment of the invention the casing is lockable in the use position and preferably also in the storage position by means of interacting members on the casing and on the socket.

The socket making part of the device according to the invention is adapted to receive an aerosol container. It should also allow passage of inhalation air along the sides of the aerosol container. Within the scope of the invention it is possible to vary the shape of the socket, which thus may have e.g. a round or oval cross-section. According to an especially preferred embodiment the socket has a rectangular, preferably a square cross-section. Thereby the socket will have two planar lateral surfaces on each side of the surface where the discharge tube is placed. The spindle means for the casing are then positioned on said lateral surfaces. With a corresponding design of the casing, where the lateral portions provided with spindle means are extended to plane parallel lateral walls, an improved tightening in the storage position and an improved control of the movement of the casing are achieved.

The device of the present invention is further described with reference to the enclosed drawings, in which

Fig. 1 shows a side view of a device according to an embodiment of the invention, in the use position, provided with an aerosol container,

Fig. 2 shows a side view of the device according to Fig. 1 in the storage position,

Fig. 3 shows a view from above of a socket making part of the device according to Fig. 1,

Fig. 4 shows a section along the line IV—IV through the socket in Fig. 3,

Fig. 5 shows a view of a casing making part of the device according to Fig. 1, and

Fig. 6 and Fig. 7 illustrate how a device according to the invention may be held in hand on dispensing of an aerosol.

In the drawings 1 denotes a socket. The socket consists of a hollow member having substantially square cross-section, the upper end 2 of which member is open and may receive an aerosol container 3, and the lower closed end 4 of which is provided with an internal spraying nozzle 5. A dosage valve in the aerosol container opens into a tube fitted into the spraying nozzle. In front of and in line with the obliquely upwardly directed orifice of the spray nozzle a discharge tube 6 is arranged, designed to be introduceable into the nostril of a patient. Two opposite lateral walls of the socket 1 have, as spindle means, two studs 7 placed substantially at the centre of said lateral walls. Said studs fit into two holes on a casing 9 to the formation of a joint, around which the casing is turnable between the use position and the storage position. The lower edge 10 of the front wall of the socket 1 is slightly extended in the forward direction in order to improve the tightening against the casing 9. The diagonally opposite edge 11 is extended to such extent that it cannot be introduced into the casing. The edge 11 thus limits the mutual movability of the casing and the socket so that only the closed end of the socket may be swung through the casing. The wall 12 of the casing is shaped like a rounded shell, which continues into two planar lateral walls wherein the holes 8 are made. An inwardly directed projection 13 on each lateral wall of the casing fits into two corresponding cavities 14 and 15 on each of the lateral walls of the socket to obtain a releasable locking of the casing in the use position and the storage position of the device respectively. The cover finally has a planar support edge 16 and a concave wall section 17, which facilitate the handling of the device.

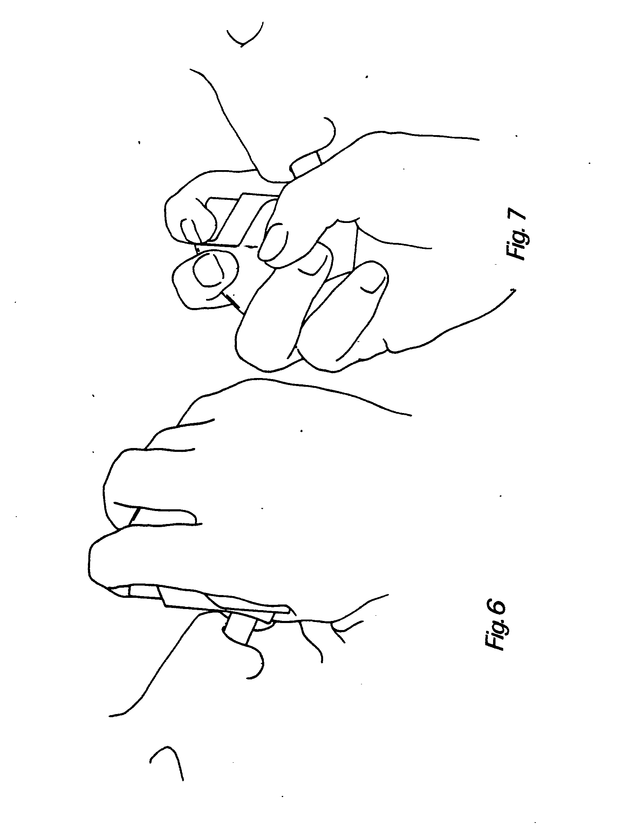

The device according to the invention may be carried locked in its storage position in the patient's pocket, handbag or the like. On use the device is held by the casing 9. By pressing the closed end of the socket said socket is easily rotated through the casing and then locked in the use position. The device is held in hand, suitably as shown in Figs. 6 or 7, and the discharge tube is introduced into the patient's nostril whereafter the aerosol container is depressed to release a dosage of drug. Concurrently with said releasing the patient may inhale through the device. After use the device is returned to the storage position.

The device of the invention may preferably be made to be disposed of after consumption of one aerosol container. In the alternative, the aerosol container may be exchangeable. The device is suitably provided with the aerosol container placed in the socket.

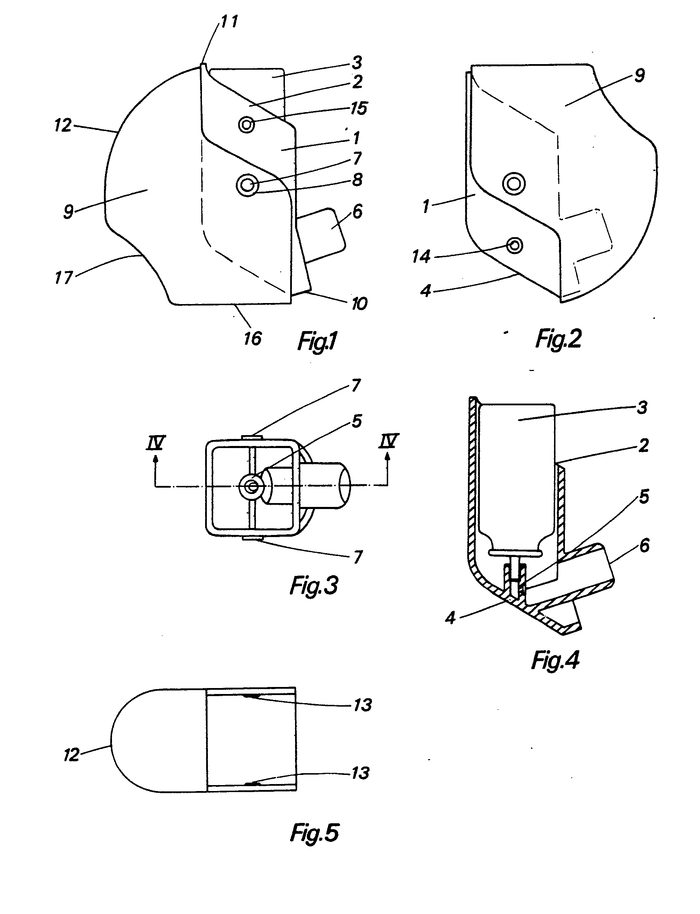

The device according to the invention has a very compact design and may be hidden in the hand on use. The discreet use thus made possible has been found desirable to many patients. The compact design is enabled by the central positioning of the spindle means. In the device according to Fig. 1 the axis of the spindle means is thus close to a point half-way between the edges 10 and 11 of the socket, and a point half-way between the corresponding edges of the casing. Within the scope of the invention it is possible to allow the axis of the spindle means to deviate somewhat from said points. With increasing distance, however, the objects of the invention are achieved to a decreasing degree.

Further, one may alternatively design the device thus that the open end of the socket, having the aerosol container held therein, may be swung through the casing.

A material for manufacture of the device is suitably a plastic material which may be shaped in a manner known *per se*. The casing can suitably be made of transparent material, rendering possible text on the aerosol container readable therethrough.

Best mode of carrying out the invention

The embodiment shown in the drawings is preferred, and it is suitably prepared by injection moulding of polypropylene for the socket and styrene acrylonitrile (SAN) for the casing.

**Claims**

1. An aerosol dispensing device comprising a socket (1), said socket having an open end (2) opening across the longitudinal direction thereof and being adapted to receive and hold an aerosol container (3); said socket being closed at the opposite end (4) thereof and having, in the interior thereof, a spraying nozzle (5) adapted to interact with a dosage valve on the aerosol container, and further having a discharge tube (6) for an aerosol; said socket having attached thereto a protective member movable between a use position for the device and a storage position, at which the protective member covers the discharge tube as well as the open end of the socket and the aerosol container held therein; the protective member consisting of a casing (9) suspended to rotate about an axis transverse to the longitudinal direction of the socket, characterised in that said casing (9) is suspended on two spindle means (7) having a common axis extending through a central part of the socket, close to a point half-way between the edges (10, 11) of the closed end and the open end of the socket, and close to a point half-way between the corresponding edges of the casing (9).

2. An aerosol dispensing device according to claim 1, characterised in that the casing (9) is lockable in the use position by means of interacting members (13, 14) on the casing and on the socket.

3. An aerosol dispensing device according to claim 1 or claim 2, characterised in that the casing is lockable in the storage position by means of interacting members (13, 15) on the casing and on the socket.

4. An aerosol dispensing device according to one or more of the preceding claims, characterised in that the socket (1) has a rectangular cross-section and that the casing (9) is suspended by means of spindle means (7) on two opposite lateral surfaces of the socket adjacent to the surface where the discharge tube (6) is placed.

5. An aerosol dispensing device according to one or more of the preceding claims characterized in that the casing (9) is suspended in such manner that in the use position it substantially covers the rear socket surface opposite to the surface where the discharge tube (6) is placed.

**Patentansprüche**

1. Aerosolabgabevorrichtung umfassend eine Hülse (1), wobei die Hülse ein sich über ihre Längsrichtung öffnendes offenes Ende (2) aufweist und so gestaltet ist, daß sie einen Aerosolbehälter (3) aufnehmen und halten kann; wobei die Hülse am gegenüberliegenden Ende (4) geschlossen ist und in ihrem Inneren eine Sprühdüse (5) aufweist, die so gestaltet ist, daß sie mit einem Dosierungsventil auf dem Aerosolbehälter zusammenwirkt, und weiter ein Abgaberohr (6) für ein Aerosol aufweist; wobei an der Hülse ein Schutzteil befestigt ist, der zwischen einer Verwendungsposition für die Vorrichtung und einer Lagerposition, in welcher der Schutzteil sowohl das Abgaberohr als auch das offene Ende der Hülse und den darin gehalterten Aerosolbehälter bedeckt, bewegbar ist; und wobei der Schutzteil aus einem Gehäuse (9) besteht, das zur Drehung um eine Achse quer zur Längsrichtung der Hülse aufgehängt ist, dadurch gekennzeichnet, daß das Gehäuse (9) auf zwei Zapfeneinrichtungen (7) mit einer gemeinsamen, sich durch einen mittleren Teil der Hülse erstreckenden Achse, nahe einem Punkt, der nahe der Mitte zwischen den Kanten (10, 11) des geschlossenen Endes und des offenen Endes der Hülse liegt, und nahe einem Punkt, der in der Mitte zwischen den entsprechenden Kanten des Gehäuses (9) liegt, aufgehängt ist.

2. Aerosolabgabevorrichtung nach Anspruch 1, dadurch gekennzeichnet, daß das Gehäuse (9) in der Verwendungsposition mittels zusammenwirkender Teile (13, 14) auf dem Gehäuse und auf der Hülse verriegelbar ist.

3. Aerosolabgabevorrichtung nach Anspruch 1 oder Anspruch 2, dadurch gekennzeichnet, daß das Gehäuse in der Lagerposition mittels zusammenwirkender Teile (13, 15) auf dem Gehäuse und auf der Hülse verriegelbar ist.

4. Aerosolabgabevorrichtung nach einem oder mehreren der vorhergehenden Ansprüche, dadurch gekennzeichnet, daß die Hülse (1) einen rechteckigen Querschnitt hat und daß das Gehäuse (9) mittels Zapfeneinrichtungen (7) an zwei gegenüberliegenden seitlichen Oberflächen der Hülse neben der Oberfläche, an welcher das Abgaberohr (6) angeordnet ist, aufgehängt ist.

5. Aerosolabgabevorrichtung nach einem oder mehreren der vorhergehenden Ansprüche, dadurch gekennzeichnet, daß das Gehäuse (9) so aufgehängt ist, daß es in der Verwendungsposition im wesentlichen die hintere Hülsenoberfläche gegenüber der Oberfläche, an welcher das Abgaberohr (6) angeordnet ist, bedeckt.

**Revendications**

1. Un dispositif distributeur pour aérosols comprenant une douille (1), ladite douille ayant une extrémité ouverte (2) débouchant transversalement à la direction longitudinale de celle-ci et étant adaptée pour recevoir et maintenir un récipient à aérosol (3); ladite douille étant fermée à l'extrémité opposée (4) de celle-ci et comportant, à l'intérieur, une buse de pulvérisation (5) adaptée pour coopérer avec une soupape de dosage sur le récipient à aérosol, et comportant de plus un tube de décharge (6) pour un aérosol; un élément de protection étant fixé à ladite douille et pouvant être déplacé entre une position d'utilisation pour le dispositif et une position de stockage, dans laquelle l'élément de protection recouvre le tube de décharge, de même que l'extrémité ouverte de la douille et du récipient à aérosol qu'elle contient, l'élément de protection étant constitué d'un boîtier (9) suspendu à rotation autour d'un axe transversal à la direction longitudinale de la douille, caractérisé en ce que ledit boîtier (9) est suspendu sur deux moyens d'articulation (7) ayant un axe commun s'étendant en travers une partie centrale de la douille, tout près d'un point, à mi-chemin entre les bords (10, 11) de l'extrémité fermée et de l'extrémité ouverte de la douille, et tout près d'un point situé à mi-chemin entre les bords correspondants du boîtier (9).

2. Un dispositif de distribution d'aérosol selon la revendication 1, caractérisé en ce que le boîtier (9) est verrouillable dans la position d'utilisation, par l'intermédiaire d'éléments coopérant (13, 14) sur le boîtier et sur la douille.

3. Un dispositif de distribution d'aérosol, selon l'une des revendications 1 ou 2, caractérisé en ce que le boîtier est verrouillable en position de stockage, par l'intermédiaire de deux éléments coopérant (13, 15) sur le boîtier et sur la douille.

4. Un dispositif de distribution d'aérosol selon l'une ou plusieurs des revendications précédentes, caractérisé en ce que la douille (1) présente une section transversale rectangulaire et que le boîtier (9) est suspendu par l'intermédiaire de moyens d'articulation (7) sur les deux surfaces latérales opposées de la douille, adjacentes à la surface où le tube de décharge (6) est placé.

5. Un dispositif de distribution d'aérosol selon l'une ou plusieurs des revendications précédentes, caractérisé en ce que le boîtier (9) est suspendu de telle sorte que en position d'utilisation, il couvre essentiellement la surface arrière de la douille opposée à la surface où le tube de décharge (6) est placé.

Fig.1

Fig.2

Fig.3

Fig.4

Fig.5

Fig. 7

Fig. 6